# FASCICULE DE BREVET EUROPEEN

(11) **EP 0 568 463 B1**
(45) Date de publication et mention de la délivrance du brevet: **04.08.1999**
(21) Numéro de dépôt: 93401134.7
(22) Date de dépôt: 30.04.1993
(51) Int. Cl.: A61N 1/05

(54) **Sonde pour stimulateur cardiaque**
Sonde für Herzschrittmacher
Pacemaker lead

(30) Priorité: 30.04.1992 FR 9205356
(43) Date de publication de la demande: 03.11.1993
(73) Titulaire: ELA MEDICAL (Société anonyme), F-92541 Montrouge Cédex (FR)
(72) Inventeur: Franchi, Pierre, F-94400 Vitry/Seine (FR)
(74) Mandataire: Laget, Jean-Loup

(56) Documents cités:
- US-A- 3 978 865
- US-A- 4 011 872
- US-A- 4 144 890
- US-A- 4 953 564
- NTIS TECH NOTES Décembre 1990, SPRINGFIELD,VA,US page 1121 COLLINS 'Implantable electrode for critical locations'

## Description

L'invention concerne une sonde pour stimulateur cardiaque.

Une telle sonde se compose d'un cathéter à l'extrémité distale duquel est disposé un capteur à plusieurs électrodes, par exemple au moins une électrode de détection et une électrode de stimulation.

Usuellement, cette extrémité distale est fixée à la paroi cardiaque par un dispositif d'accrochage du type à barbules ou à vis, de façon que les électrodes de stimulation et de détection soient maintenues contre la paroi cardiaque. Les électrodes sont généralement en forme de disque ou d'anneau, afin de présenter la plus grande partie possible de leur surface en contact avec la paroi cardiaque.

Dans certaines applications, les électrodes de détection sont distinctes des électrodes de stimulation et ne sont pas en contact avec la paroi cardiaque.

Dans le document US 4 365 639, les électrodes de détection sont disposées sur la surface latérale du cathéter, à distance de l'électrode de stimulation.

Dans le document EP 0 191 238, l'électrode de stimulation est en forme de disque et les électrodes de détection sont en forme de tige dont l'extrémité sphérique est appliquée sur la paroi cardiaque à travers une ouverture de l'électrode de stimulation.

Dans chacun des deux cas précédents, la partie d'extrémité distale de la sonde cardiaque est pratiquement rigide. Lors de la fixation de la sonde à la paroi cardiaque, l'objectif recherché est d'assurer un bon contact entre l'électrode de stimulation et la paroi cardiaque. C'est pourquoi la fixation de la sonde est assurée de manière efficace, c'est-à-dire traumatisante. Or, la création de fibrine et la lésion de la paroi cardiaque qui altère les cellules, entraînent une dégradation de la stimulation aussi bien que de la détection. Ce phénomène est aggravé par les efforts transmis par le cathéter à la fixation au cours des battements du coeur. A titre d'exemple, la sonde à vis du document EP 0 191 238 crée une forte lésion.

Un premier but de l'invention et de proposer une sonde pour stimulateur cardiaque susceptible d'être fixée à la paroi cardiaque sans lésion au droit de l'électrode, de façon a séparer le site de l'activation cardiaque du site de la lésion.

Un autre but de la présente invention est de proposer une sonde pour stimulateur cardiaque, susceptible d'être mise en place pratiquement sans lésion, de façon à être facilement déplacée en cas de défaut de positionnement, ou si les tests à l'implantation ne sont pas satisfaisants.

Un autre but de l'invention est de proposer une sonde pour stimulateur cardiaque dont le capteur d'extrémité ne soit pas rigidement fixé au cathéter de la sonde de façon à le découpler mécaniquement, de façon à permettre et favoriser l'application, dans la meilleure position, du capteur sur la paroi cardiaque, et de façon à maintenir, avec le minimum de contraintes et en position relative stable, le capteur en contact intime avec la paroi cardiaque, indépendamment des mouvements du coeur.

L'invention a pour objet une sonde pour stimulateur cardiaque, du type comportant un capteur des signaux cardiaques,pouvant être mis en place dans un ventricule aussi bien que dans une oreillette, portant des électrodes, susceptible d'appliquer au coeur des signaux de stimulation, et un cathéter disposé entre ledit capteur et le boîtier du stimulateur, caractérisée en ce que la sonde comporte un cordon souple de raccordement du capteur au cathéter dans lequel passent les liaisons électriques vers les électrodes, en ce que le système de fixation du cathéter au capteur est réversible , le capteur étant désolidarisable du cathéter, et, en ce que, après sa fixation au myocarde, le capteur est relié au cathéter par le cordon souple.

Selon d'autre caractéristiques de l'invention :
- le capteur comporte essentiellement une plaquette isolante portant d'un côté des électrodes et de l'autre côté un bloc de raccordement au cordon souple ;
- le capteur porte des moyens d'accrochage à la paroi cardiaque répartis dessous et de préférence à la périphérie de la plaquette isolante ;
- les moyens d'accrochage du capteur à la paroi cardiaque sont des griffes inclinées, et sont mis en oeuvre par rotation du capteur ;
- les moyens d'accrochage du capteur sont des griffes inclinées situées dans des plans radiaux par rapport au capteur, et sont mis en oeuvre par déformation de la plaquette isolante ;
- le cathéter porte des moyens d'accrochage à la paroi cardiaque ;
- lesdits moyens d'accrochage du cathéter sont mis en oeuvre par déplacement relatif par rapport au cathéter ;
- la plaquette isolante présente des commissures;
- la plaquette isolante porte, autour des électrodes une substance anti-inflammatoire à diffusion lente.

D'autres caractéristiques ressortent de la description qui suit faite avec référence aux dessins annexés sur lesquels on peut voir :
Fig 1 : une vue en coupe d'une exemple de réalisation d'un capteur pour sonde de stimulateur cardiaque selon l'invention ;
Fig 2 et 3 : des variantes de réalisation du capteur de la Fig. 1 ;
Fig 4 : une vue de dessus montrant le système de fixation du capteur selon l'invention ;
Fig 5 : une vue de côté du capteur de la Fig 4 ;
Fig 6 : une vue de dessous d'une variante de réalisation du système de fixation du capteur de la Fig 4 ;
Fig 7 : une vue de côté du support d'électrodes de la Fig 6 en position de repos ;
Fig 8 : une vue de côté du support d'électrodes de la Fig 7 en position de mise en place ;
Fig 9 : une vue de côté d'une variante de réalisation du support d'électrodes de la Fig 6 en position de repos ;
Fig 10 : une vue du support d'électrodes de la Fig 9 en position de mise en place ;
Fig 11 : une vue en coupe d'un capteur selon l'invention ;
Fig 12 : une vue partielle de dessus du capteur de la Fig 11 ;
Fig 13 : une vue du capteur selon l'invention dans son cathéter ;
Fig 14 : une vue en coupe montrant l'application du capteur sur le myocarde au moment de la mise en place ;
Fig 15 : une vue en coupe montrant la disposition du cathéter et du capteur de la Fig 14, en position d'utilisation ;
Fig 16 : une vue d'un exemple de réalisation du système de fixation du cathéter, en position repliée ;
Fig 17 : une vue du système de fixation de la Fig 16 en position déployée ;
Fig 18 : une vue d'un autre exemple de réalisation du système de fixation du cathéter en position de mise en place ;
Fig 19 : une vue du système de fixation de la Fig 18 en position d'utilisation.

Selon l'invention, la sonde pour stimulateur cardiaque se compose d'un cathéter et d'un capteur. Le capteur est destiné à être fixé sur la paroi cardiaque. Il est placé à l'extrémité distale du cathéter, et il comprend essentiellement : une feuille ou plaquette isolante jouant le rôle de support d'électrodes, des électrodes destinées à être appliquées sur la paroi cardiaque, et des moyens de fixation du support d'électrodes à la paroi cardiaque.

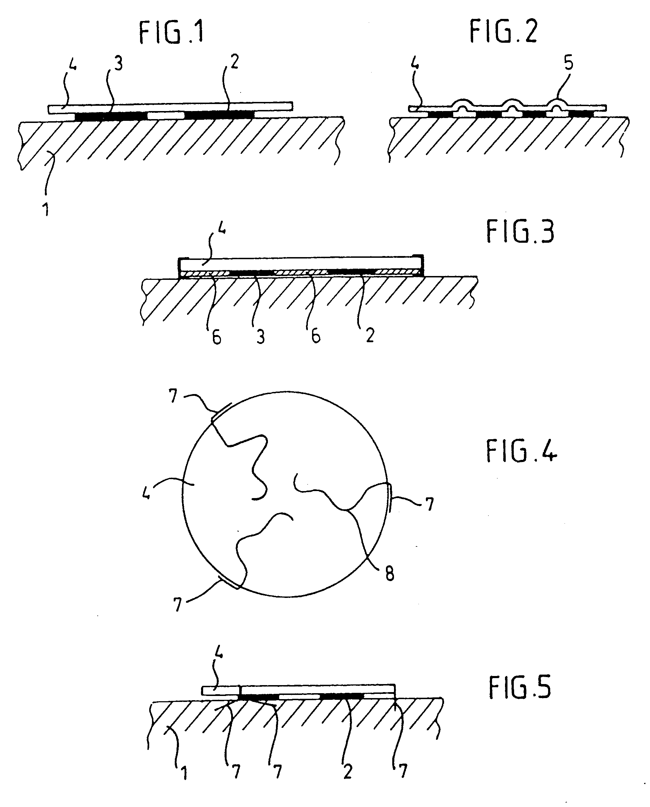

Sur la Fig 1, la paroi cardiaque (myocarde) est symbolisée en 1, les électrodes en 2 et 3, et la feuille isolante de support des électrodes en 4. La feuille isolante 4 peut être en matériau souple, par exemple en matière plastique. L'ensemble du capteur est léger et de faible encombrement. De ce fait, le capteur respecte mieux l'intégrité du muscle cardiaque au contact duquel il se trouve. La couche de fibrine qui se forme autour du capteur par frottement, pression ou traction sur le muscle au cours des mouvements du coeur est par conséquent de faible épaisseur et altère peu le fonctionnement du capteur.

Sur la Fig 2, la feuille isolante 4 présente des commissures linéaires telles que 5, placées perpendiculairement à la direction de la contraction, pour donner de la souplesse à l'ensemble du capteur.

Sur la Fig 3, la feuille isolante 4 porte, autour des électrodes 2, 3, une substance 6 anti-inflammatoire à dilution lente, ayant pour objet de réduire la formation de fibrine.

La diffusion de la substance anti-inflammatoire est lente d'une part parce qu'elle peut résulter d'une libération progressive, mais aussi, d'autre part, parce que la substance se trouve dans un espace confiné où le sang ne circule pas ou ne circule que très peu. Cela est très différent du cas des cathéters usuels avec électrodes, qui sont balayés par le flux sanguin.

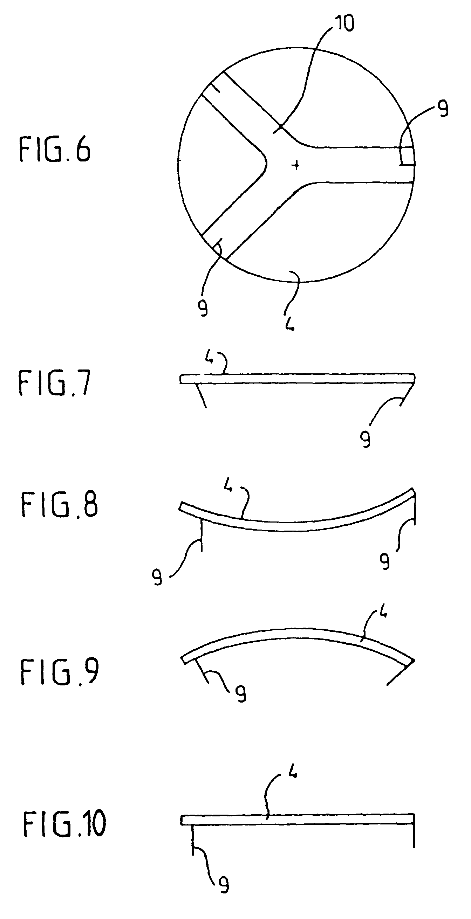

Le système de fixation du capteur sur le myocarde comporte essentiellement des griffes 7, par exemple au nombre de 3 sur la Fig. 4, réparties à la périphérie du capteur, tangentiellement au capteur, et faisant un angle avec le plan de la feuille 4. Cet angle, de l'ordre de 30°, est visible sur la Fig 5. Chaque griffe 7 est prolongée par un corps 8 d'ancrage, de forme appropriée à sa fonction, et situé de préférence dans la feuille 4 de support des électrodes. Le corps 8 d'ancrage présente au moins deux parties faisant un angle entre elles et situées toutes deux dans la feuille 4, de façon à assurer le blocage en position de la griffe 7. Le corps d'ancrage 8 peut présenter un profil courbe dans la feuille 4 de façon à lui donner plus de rigidité au moment de la fixation du capteur sur le myocarde. Les griffes 7 peuvent avantageusement être courbes, ce qui favorise leur pénétration en rotation. Pour assurer leur maintien dans la paroi cardiaque, elles sont de préférence pourvues d'une rugosité faible à la pénétration et forte à l'extraction. Le capteur selon les Fig 4 et 5 est mis en place par une petite rotation autour de son axe, rotation au cours de laquelle les griffes 7 pénètrent dans le myocarde.

La fixation du capteur au myocarde peut être assurée, de façon supplétive ou additionnelle, par des barbules très fines, réparties à la périphérie de la feuille 4, de façon à remplacer les griffes 7 ou compléter leur action. La croissance de la fibrine qui emprisonne les barbules assure la fixation dans le temps du capteur sur le tissu cardiaque. De préférence, les barbules sont réalisées en matériau bio-résorbable. Elles peuvent être constituées en tissu synthétique, par exemple à base de polyéthylène-téréphtalates, connu sous le nom de Dacron.

Sur la Fig. 6, les griffes 9 sont réparties à la périphérie de la feuille 4, et chaque griffe 9 est située dans un plan radial contenant l'axe du capteur. Dans ce plan, chaque griffe est inclinée sur la feuille 4 d'un angle de l'ordre de 70°, par exemple. Ainsi, lorsque la feuille 4 est au repos, c'est-à-dire plane, les griffes sont inclinées par rapport à son axe (axe vertical sur la Fig 7), et lorsque la feuille 4 est cintrée pour la mise en place du capteur sur la paroi cardiaque, les griffes 9 sont parallèles à l'axe du capteur (axe vertical sur la Fig 8) afin de pouvoir pénétrer sans difficulté dans le myocarde, après quoi, l'élasticité de la feuille 4 ramène l'ensemble dans la position de la Fig 7.

Pour assurer l'élasticité de la feuille 4, il est possible de prévoir, sur la feuille 4, une pièce métallique faisant office de ressort. Cette pièce métallique peut avoir une forme étoilée comme représentée en 10 sur la Fig 6.

La feuille 4 peut être cintrée en position de repos (Fig 9) et plate en position de mise en place (Fig 10).

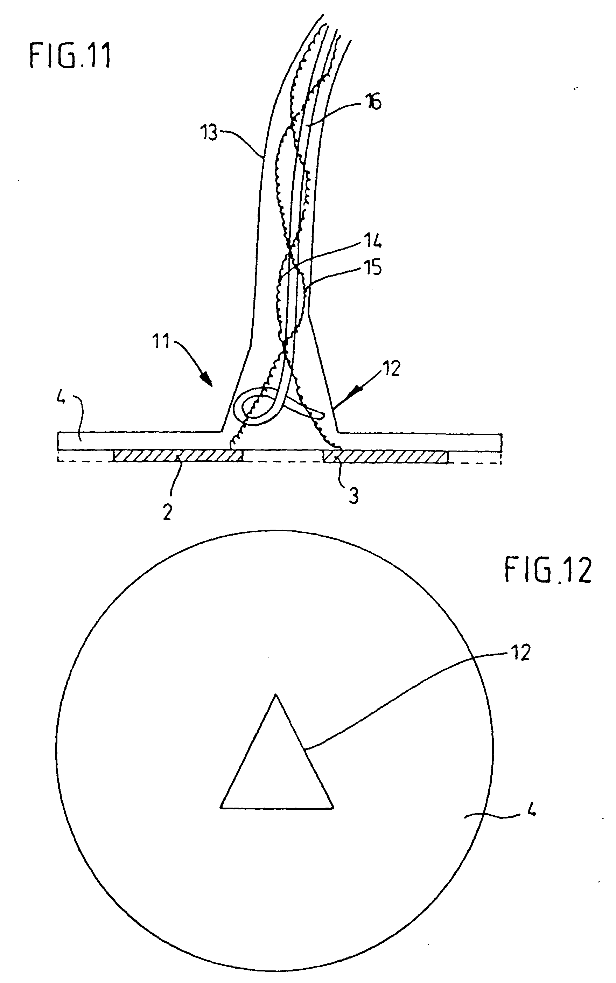

Sur la Fig 11 est représenté en coupe le capteur 11 de la sonde selon l'invention. Ce capteur se compose de la feuille 4 de support des électrodes (les griffes de fixation ne sont pas représentées), des électrodes 2, 3, d'un bloc de raccordement 12 disposé au dos de la feuille 4, et d'un cordon souple 13 à l'intérieur duquel sont disposés des conducteurs électriques 14, 15 de liaison aux électrodes, et un cordon 16 de reprise d'effort.

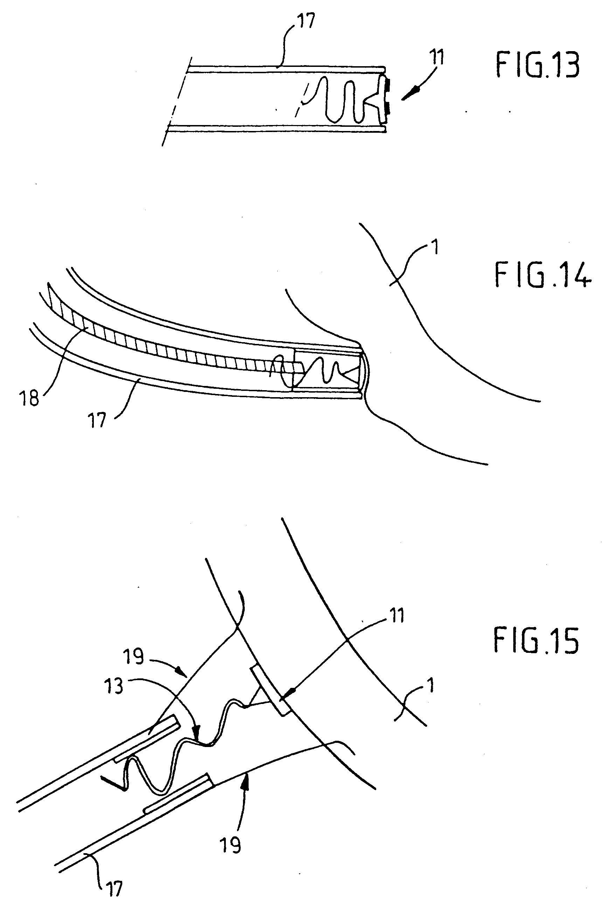

Le bloc de raccordement 12 est, par exemple, de forme pyramidale pour recevoir un outil capable d'effectuer les manoeuvres de rotation correspondant au verrouillage et au déverrouillage des griffes de fixation. Le bloc de raccordement 12, pour la manoeuvre du capteur, peut être remplacé par des reliefs tels que des ergots plats, des protubérances ou des retraits, répartis à la périphérie de la feuille 4. Pour la mise en place du capteur sur le tissu cardiaque, le cathéter 17 (Fig. 13) peut présenter des formes complémentaires des reliefs portés par la feuille 4.

Les conducteurs électriques 14, 15 sont de préférence constitués par des fils d'acier, de titane ou de platine, finement spiralés. Le cordon de reprise d'effort 16 est par exemple en matière plastique de haute résistance, et ancré dans le bloc de raccordement 12. Le cordon souple 13 est de préférence revêtu de silicone ou de polyuréthanne, soit par apport d'une gaine, soit par surmoulage.

La sonde selon l'invention se compose d'un cathéter 17 et du capteur 11 (Fig 13). A l'intérieur du cathéter 17, un mandrin 18 permet de guider la sonde cardiaque jusqu'à l'appliquer avec force contre le myocarde 1 (Fig 14) quelle que soit la forme du myocarde en regard du capteur 11, et quelle que soit la position de la surface du myocarde par rapport à l'axe du cathéter.

Le capteur 11 est alors fixé au myocarde, soit par les griffes 7, par rotation du capteur 11 commandée par le mandrin 18, soit par les griffes 9 par déformation élastique du capteur 11.

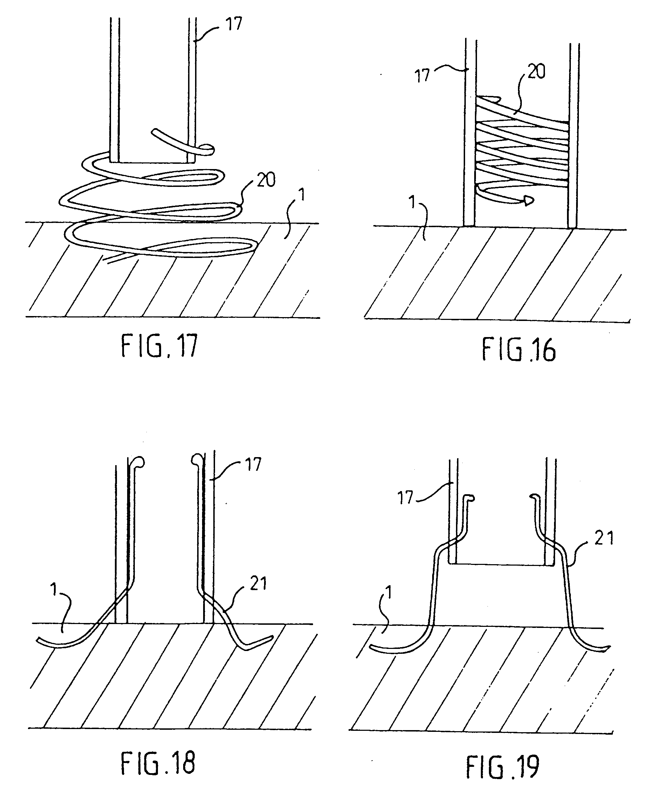

Le cathéter est fixé au myocarde par une fixation semi-rigide 19 (Fig 15). Le capteur 11 est désolidarisé du cathéter 17, et le cathéter est tiré à distance du myocarde 1, puis maintenu dans cette position éloignée par la fixation semi-rigide 19.

Le cordon souple 13 de raccordement du capteur 11 au cathéter 17, reste libre et non tendu. Cette disposition présente l'avantage de maintenir le capteur appliqué à la surface du myocarde sans contrainte mécanique de la part du cathéter. De ce fait, les électrodes sont préservées de toute contrainte mécanique ce qui préserve les performances du capteur et les propriétés électrophysiologiques du myocarde.

La fixation semi-rigide du cathéter 17 peut être constituée par un ressort hélicoïdal 20 (Fig 16) simple ou multiple, extrait du cathéter et se développant sur un diamètre supérieur à celui du cathéter (Fig 17). Elle peut aussi être constituée par un groupe de bras 21 qui sont ancrés dans le myocarde 1 lorsque le cathéter 17 est en appui sur le myocarde (Fig 18), qui sont ensuite déployés lors du retrait du cathéter, et qui sont enfin verrouillés par rapport au cathéter 17 dans la position retirée du cathéter (Fig 19).

Le système de fixation du cathéter 17 et du capteur 11 est réversible. Par traction sur la partie des bras 21 interne au cathéter 17 (Fig 19) ou par rotation du ressort 20 (Fig 17), le cathéter 17 se rapproche du myocarde, le cordon souple 13 se replace à l'intérieur du cathéter 17, le cathéter se recentre grâce au bloc de raccordement 12 du capteur 11, et le capteur est retiré du myocarde. Si un obstacle s'opposait à cette dernière manoeuvre, le capteur pourrait être ramené par traction sur le cordon souple 13 de raccordement, dont la résistance est supérieure à la force d'arrachement du capteur maintenu par les griffes et/ou les lambeaux de fibrine.

La sonde selon l'invention présente l'avantage de mettre en place les électrodes, portées par le capteur, pratiquement sans lésion, et de pouvoir les retirer en cas de défaut de positionnement, ou si les tests à l'implantation ne sont pas satisfaisants, ou ultérieurement, s'il y a nécessité de retirer le capteur pour cause d'infection, ou de le déplacer parce que la zone où il se trouve appliqué a perdu son activité, par exemple en cas d'infarctus. De plus, le capteur est de dimensions très restreintes, et il peut être mis en place dans un ventricule aussi bien que dans une oreillette, pratiquement sans lésion du myocarde.

De préférence, les électrodes sont des plaques conductrices minces et non pesantes, réalisées par exemple sous forme de dépôt de métal ou de carbone.

Un avantage du capteur selon l'invention est qu'il peut porter plus de deux électrodes. Par exemple, il peut être envisagé d'utiliser, sur le même capteur, au moins une électrode de détection et deux électrodes de stimulation. Il en résulte que l'énergie de seuil de l'impulsion de stimulation sera plus faible pour dépolariser les cellules du coeur afin de déclencher une contraction.

## Revendications

1. Sonde pour stimulateur cardiaque, du type comportant un capteur des signaux cardiaques,pouvant être mis en place dans un ventricule aussi bien que dans une oreillette, portant des électrodes, susceptible d'appliquer au coeur des signaux de stimulation, et un cathéter disposé entre ledit capteur et le boîtier du stimulateur, caractérisée en ce que la sonde comporte un cordon souple (13) de raccordement du capteur (11) au cathéter (17) dans lequel passent les liaisons électriques vers les électrodes, en ce que le système de fixation du cathéter (17) au capteur (11) est réversible , le capteur (11) étant désolidarisable du cathéter (17), et en ce que, après sa fixation au myocarde, le capteur (11) est relié au cathéter (17 par le cordon souple (13)

2. Sonde selon la revendication 1, caractérisée en ce que ledit capteur comporte essentiellement une plaquette isolante (4) portant d'un côté des électrodes (2, 3) et de l'autre côté un bloc (12) de raccordement au cordon souple (13).

3. Sonde selon la revendication 1, caractérisée en ce que le capteur (11) porte des moyens d'accrochage à la paroi cardiaque répartis dessous et de préférence à la périphérie de la plaquette isolante (4).

4. Sonde selon la revendication 3, caractérisée en ce que les moyens d'accrochage du capteur (11) à la paroi cardiaque sont des griffes (7) inclinées, et sont mis en oeuvre par rotation du capteur.

5. Sonde selon la revendication 3, caractérisée en ce que les moyens d'accrochage du capteur (11) sont des griffes (9) inclinées situées dans des plans radiaux par rapport au capteur, et sont mis en oeuvre par déformation de la plaquette isolante (4).

6. Sonde selon la revendication 1, caractérisée en ce que le cathéter (17) porte des moyens d'accrochage à la paroi cardiaque.

7. Sonde selon la revendication 6, caractérisée en ce que lesdits moyens (20, 21) d'accrochage du cathéter (17) sont mis en oeuvre par déplacement relatif par rapport au cathéter.

8. Sonde selon la revendication 1, caractérisée en ce que la plaquette isolante (4) présente des commissures (5).

9. Sonde selon la revendication 1, caractérisée en ce que la plaquette isolante (4) porte, autour des électrodes (2, 3) une substance anti-inflammatoire à diffusion lente (6).

## Claims

1. A probe for cardiac pacemaker of the type comprising a heart signal sensor that can be implanted equally well in a ventricle or in an auricle, which sensor comprises electrodes and is susceptible of applying pacing signals to the heart, and a catheter disposed between said sensor and the pacemaker box, characterised in that the probe is provided with a flexible cord (13) for attaching the sensor (11) to the catheter (17) and through which the electric connections lead to the electrodes, in that the system for attaching the catheter (17) to the sensor (11) is reversible, the sensor (11) being detachable from the catheter (17), and in that the sensor (11), after having been attached to the myocardium, is connected to the catheter (17) by the flexible cord (13).

2. The probe as claimed in claim 1, characterised in that said sensor mainly comprises a insulating board (4) comprising on one side said electrodes (2, 3) and on the other side a block (12) for connection to said flexible cord (13).

3. The probe as claimed in claim 1, characterised in that said sensor (11) comprises means for catching to said cardiac wall distributed over the underside and preferably around the periphery of said insulating board (4).

4. The probe as claimed in claim 3, characterised in that said means for catching the sensor (11) to the cardiac wall are angled claws (7) and are installed by rotation of said sensor.

5. The probe as claimed in claim 3, characterised in that said means for catching the sensor (11) are angled claws (9) situated in radial planes containing the axis of said sensor, and are installed by deformation of said insulating board (4).

6. The probe as claimed in claim 1, characterised in that said catheter (17) comprises means for catching to said cardiac wall.

7. The probe as claimed in claim 6, characterised in that said means (20, 21) for catching the catheter (17) are installed by relative displacement in relation to said catheter.

8. The probe as claimed in claim 1, characterised in that said insulating board (4) has commissures (5).

9. The probe as claimed in claim 1, characterised in that said insulating board (4) has a slow-diffusion anti-inflammatory substance (6) around said electrodes (2, 3).

## Patentansprüche

1. Sonde für einen Herzschrittmacher von einer Bauart, die ein Signalglied für Herzsignale, das sowohl in einer Herzkammer als auch in einem Herzvorhof angeordnet werden kann, Elektroden trägt, Stimulationssignale auf das Herz aufbringen kann, und einen Katheter aufweist, der zwischen dem Signalglied und dem Gehäuse des Schrittmacher angeordnet ist, **dadurch gekennzeichnet, daß** die Sonde eine biegsame Leitung (13) zum Anschluß des Signalglieds (11) an den Katheter (17) umfaßt, in der elektrische Leitungen zu den Elektroden verlaufen, das System zur Befestigung des Katheters (17) an dem Signalglied (11) reversibel ist, wobei das Signalglied (11) von dem Katheter (17) abtrennbar ist, und das Signalglied (11) nach seiner Befestigung an dem Herzmuskel mit dem Katheter (17) durch die biegsame Leitung (13) verbunden ist.

2. Sonde nach Anspruch 1,
**dadurch gekennzeichnet, daß**
das Signalglied im wesentlichen ein Isolierplättchen (4) umfaßt, das auf einer Seite Elektroden (2, 3) und auf der anderen Seite einen Block (12) zum Anschluß an die biegsame Leitung (13) trägt.

3. Sonde nach Anspruch 1,
**dadurch gekennzeichnet, daß**
das Signalglied (11) Mittel zum Festklemmen an der Herzwand trägt, die unter und bevorzugt an dem Rand des Isolierplättchens (4) verteilt sind.

4. Sonde nach Anspruch 3,
**dadurch gekennzeichnet, daß**
die Mittel zum Festklemmen des Signalglieds (11) an der Herzwand geneigte Klauen (7) sind, die durch Drehung des Signalglieds eingesetzt werden.

5. Sonde nach Anspruch 3,
**dadurch gekennzeichnet, daß**
die Mittel zum Festklemmen des Signalglieds (11) geneigte Klauen (9) sind, die in Bezug zu dem Signalglied in radialen Ebenen liegen und die durch Verformung des Isolierplättchens (4) eingesetzt werden.

6. Sonde nach Anspruch 1,
**dadurch gekennzeichnet, daß**
der Katheter (17) Mittel zum Festklemmen an der Herzwand trägt.

7. Sonde nach Anspruch 6,
**dadurch gekennzeichnet, daß**
die Mittel (20, 21) zum Festklemmen des Katheters (17) durch eine relative Versetzung in Bezug zu dem Katheter eingesetzt werden.

8. Sonde nach Anspruch 1,
**dadurch gekennzeichnet, daß**
das Isolierplättchen (4) Ausbeulungen (5) aufweist.

9. Sonde nach Anspruch 1,
**dadurch gekennzeichnet, daß**
das Isolierplattchen (4) um die Elektroden (2, 3) herum eine mit langsamer Verbreitung entzündungshemmende Substanz tragt.
